(19) Europäisches Patentamt
European Patent Office
Office européen des brevets

(11) **EP 0 363 894 B1**

(12) **EUROPÄISCHE PATENTSCHRIFT**

(45) Veröffentlichungstag und Bekanntmachung des Hinweises auf die Patenterteilung:
**23.07.1997 Patentblatt 1997/30**

(51) Int. Cl.$^6$: **C07D 201/16**, C07D 207/267

(21) Anmeldenummer: 89118792.4

(22) Anmeldetag: **10.10.1989**

(54) **Verfahren zur Extraktion von N-Methyl-Pyrrolidon-(2)**

Process for the extraction of N-methyl-2-pyrrolidone

Procédé d'extraction de la N-méthyl-2-pyrrolidone

(84) Benannte Vertragsstaaten:
**AT BE CH DE FR GB IT LI LU NL**

(30) Priorität: **13.10.1988 DE 3834904**

(43) Veröffentlichungstag der Anmeldung:
**18.04.1990 Patentblatt 1990/16**

(73) Patentinhaber: **HOECHST AKTIENGESELLSCHAFT**
**65926 Frankfurt am Main (DE)**

(72) Erfinder:
• **Miess, Georg-Emerich, Dr.**
**D-6240 Königstein/Taunus (DE)**
• **Schwarz, Karl-Heinz**
**D-6237 Liederbach (DE)**
• **Tetzlaff, Heribert, Dr.**
**D-6000 Frankfurt am Main 71 (DE)**
• **Wojtech, Bernhard, Dr.**
**D-6232 Bad Soden am Taunus (DE)**

(56) Entgegenhaltungen:
DE-A- 2 252 102          DE-A- 2 332 973
FR-A-   896 883          US-A- 4 013 640

• **J. CHEM. SOC., FARADAY TRANS. 1, Band 83, Nr. 9, 1987, Seiten 2867-2881; M.H. ABRAHAM et al.: "Hydrogen bonding"**

Bemerkungen:
Die Akte enthält technische Angaben, die nach dem Eingang der Anmeldung eingereicht wurden und die nicht in dieser Patentschrift enthalten sind.

**Beschreibung**

Die vorliegende Erfindung betrifft ein Verfahren zur Extraktion von N-methyl-Pyrrolidon-(2), nachfolgend NMP genannt, aus wäßrigen Lösungen.

Wäßrige NMP-Lösungen fallen in der Technik unter anderem in der Petrochemie (Aromatenextraktion aus Rohöl) und bei der Herstellung hitzebeständiger Polymerer wie Polyamide und Polyimide an. Im Stand der Technik sind im wesentlichen Destillations- und Extraktionsverfahren zur Abtrennung des NMP aus wäßrigen Lösungen bekannt. Das Aussalzen, wie in Y. Nagaosa, Anal. Chim. Acta (1980) 120, 279-87 beschrieben, ist nur von untergeordneter Bedeutung.

Die Destillationsverfahren zur Abtrennung des NMP aus wäßrigen Lösungen werden im Stand der Technik üblicherweise bei geringen Wassergehalten des NMP angewandt. Derartige Verfahren sind aus Chemical Abstracts, Vol. 70, 1969, Referat 108066k und Chemical Abstracts, Vol. 101, 1988, Referat 9842n bekannt. Je nach den Bedingungen ist auch zusätzlich oder ausschließlich ein Strip des Wassers aus dem NMP mit einem Inertgas bekannt, wie aus US-PS 4,057,491 und DE-OS 27 09 679 hervorgeht.

Extraktionsverfahren werden im Stand der Technik besonders vorteilhaft bei höheren Wassergehalten der zu behandelnden Lösungen eingesetzt. Durch Vermeidung der kostenaufwendigen rektifikativen Trennung von Wasser und NMP ergeben sich hier Kostenvorteile. Zur Wiedergewinnung des NMP ist jedoch nach der Extraktion noch eine destillative Trennung zwischen NMP und dem Extraktionsmittel erforderlich.

Die im Stand der Technik bekannten Extraktionsmittel gehören verschiedenen Verbindungsklassen an:

In JP 49/48 432 (Chemical Abstracts, Vol 82, Referat 170665f) wird die Wiedergewinnung von NMP aus Calciumchlorid-haltigen wäßrigen Lösungen durch Extraktion mit Adipinsäuredinitril, Benzonitril, Ethylnaphthalin oder Halogenkohlenwasserstoffen beschrieben.

In Chemical Abstracts, Vol. 85, 1976, Referat 142681b wird die Wiedergewinnung von N-Alkylamid-haltigen Lösungen aus wäßrigen Lösungen beschrieben, wobei diese unter Verwendung von Halogen-Kohlenwasserstoffen aus den wäßrigen Lösungen entfernt werden.

Aus JP 55 141 450 (Chemical Abstracts, Vol 94, Referat 120315m) ist die Wiedergewinnung von N-Alkylamiden unter Verwendung von Anilinen, Alkoholen und/oder substituierten Phenolen, wie kurzkettige Alkylphenole, Halogenphenole, Alkoxyphenole und Alkyl-substituierte Dihydroxybenzole bekannt. Die genannten Hydroxy-aromatischen Verbindungen weisen jedoch eine relativ hohe Wasserlöslichkeit auf, so beispielsweise m-Kresol eine Löslichkeit von 2,3 Gew.-% und 3,5-Dimethylphenol 0,49 Gew.-% bei Raumtemperatur. Diese relativ hohe Wasserlöslichkeit hat zur Folge, daß die zu behandelnden wäßrigen Abwässer mit einem relativ hohen Gehalt an Extraktionsmittel belastet sind. Demgegenüber besteht die Aufgabe der vorliegenden Erfindung darin, neue Extraktionsmittel zur Abtrennung von NMP aus wäßrigen Lösungen zur Verfügung zu stellen, die eine äußerst geringe Löslichkeit in Wasser bei Raumtemperatur und einen hohen Siedepunkt aufweisen, der eine destillative Aufarbeitung des Extraktionsmittel-NMP-Extraktes ermöglicht, der es vorzugsweise gestattet, das NMP (Siedebereich 202,5 bis 205°C) aus dem Extraktionsmittel direkt abzudestillieren und so vom Extraktionsmittel zu isolieren.

Die US-A-4,013,640 beschreibt ein Verfahren zur Extraktion aliphatischer Amide mittels Phenolen aus einer wäßrigen Lösung. Als Amide werden die Verbindungen Epsilon-Caprolactam, N-Methyl-Epsilon-Caprolactam, 1,5-Dimethyl-2-Pyrrolidon und 2-Pyrrolidon genannt. Die Verbindung N-Methyl-Pyrrolidon (2) ist nicht genannt. Das Verfahren wird für eine Konzentration von weniger als 25 Gew. % Aramid in der wäßrigen Lösung empfohlen.

Es wurde überraschenderweise gefunden, daß Phenole, die einen längerkettigen Alkylsubstituenten aufweisen, trotz ihres hydrophoben, aus der Alkylkette resultierenden Charakters und trotz der geringen Polarität sowohl allein, als auch in Kombination mit inerten Verdünnungsmitteln, sich besonders gut zur Extraktion von NMP aus Fällbädern eignen, die bei der Verspinnung aromatischer Polyamide erhalten werden.

Die vorgenannten Aufgaben und Randbedingungen werden erfüllt durch ein Verfahren zur Extraktion von N-Methyl-Pyrrolidon-(2) aus wäßrigen Lösungen, das dadurch gekennzeichnet ist, daß als Extraktionsmittel bei Raumtemperatur in Wasser schwerlösliche oder unlösliche, einfach oder mehrfach durch geradkettige oder verzweigte aliphatische Reste kernsubstituierte Phenole eingesetzt werden und die wäßrigen Ausgangslösungen mindestens 25 Gew.-%, vorzugsweise mindestens 28 Gew.-% N-Methyl-Pyrrolidon-(2) enthalten.

Unter Mehrfachsubstitution ist insbesondere eine Zwei- oder Dreifachsubstitution zu verstehen. Bevorzugt sind die einfach substituierten Phenole. Der aliphatische Substituent, oder, bei Mehrfachsubstitution einer der aliphatischen Substituenten des Phenols hat eine längere Kohlenstoffkette, d.h. er hat 4 bis 16 C-Atome, vorzugsweise 6 bis 12 C-Atome. Evtl. vorhandene weitere aliphatische Substituenten können kurz- oder längerkettig sein, d.h. 1 bis 8 C-Atome aufweisen, wobei jedoch alle aliphatischen Substituenten des Phenols gemeinsam nicht mehr als 16, vorzugsweise nicht mehr als 12 C-Atome haben.

Bevorzugt sind alkylsubstituierte Phenole wobei mindestens eine der Alkylgruppen längerkettig ist, d.h. 4 bis 16, vorzugsweise 6 bis 12 C-Atome aufweist.

Neben der längerkettigen Alkylgruppe können die erfindungsgemäß einzusetzenden Phenolderivate noch 1 oder 2 weitere Alkylgruppen tragen, die auch kürzerkettig sein können, wobei die Gesamtzahl der Kohlenstoffatome aller Alkylsubstituenten des Phenols 5 bis 16, vorzugsweise 7 bis 12 beträgt.

Im Prinzip können auch substituierte Phenole eingesetzt werden, deren aliphatische Substituenten ungesättigt sind. Wegen der chemischen Reaktivität ungesättigter aliphatischer Gruppen ist jedoch der Einsatz von substituierten Phenolen mit solchen ungesättigten Resten weniger zweckmäßig.

Um eine möglichst einfache Trennung des extrahierten NMP vom Extraktionsmittel zu ermöglichen, sollte der Siedepunkt des erfindungsgemäß eingesetzten Phenolderivats sich um mindestens 20°C, vorteilhafterweise um mindestens 50°C vom Siedebereich des NMP, der bei etwa 202-205°C liegt, unterscheiden. Ein geringerer Siedepunktsunterschied kann toleriert werden, wenn leistungsfähige Rektifikationsapparaturen bereitstehen und der erhöhte Destillationsaufwand durch andere Verfahrensvorteile (z.B. niedriger Preis des betreffenden Phenolderivats) aufgefangen wird.

Ein besonderer Vorteil des erfindungsgemäßen Verfahrens ergibt sich dann, wenn der Siedepunkt des Extraktionsmittels im angegebenen Abstand oberhalb des Siedepunkts des NMP liegt. In diesem Fall ist es möglich, bei der Aufarbeitung des Alkylphenol-NMP-Extraktes das NMP aus dem Alkylphenol direkt abzudestillieren und so vom Extraktionsmittel zu trennen. Das NMP wird im Hauptlauf rein und praktisch wasserfrei gewonnen, während das Alkylphenol im Destillationssumpf zurückbleibt. Das Alkylphenol geht anschließend wieder im Kreislauf in die Extraktionsstufe des Verfahrens zurück.

Durch Anwendung des erfindungsgemäßen Verfahrens ist es nicht erforderlich, das extrahierte NMP vollständig vom Alkylphenol abzutrennen, da die NMP-Verteilungskoeffizienten im System NMP-Wasser-Alkylphenol ausreichend hoch sind, um z.B. auch mit Alkylphenol, das noch ca. 1 Gew.-% NMP enthält, bei Raumtemperatur die geforderten Restgehalte von vorzugsweise weniger als 0,1 Gew.-% NMP im Abwasser zu erzielen.

Die Wasserlöslichkeit der erfindungsgemäß einzusetzenden Alkyl-Phenole sollte 200 ppm bei 25°C nicht überschreiten. Gemäß einer bevorzugten Ausführungsform werden Alkylsubstituierte Phenole mit einer Wasserlöslichkeit von weniger als 100 ppm, insbesondere bevorzugt weniger als 10 ppm eingesetzt.

Aus der Gruppe der oben definierten, durch aliphatische Reste substituierten Phenole werden für den erfindungsgemäßen Einsatz zweckmäßigerweise solche gewählt, deren Schmelzpunkt unter 90°C vorzugsweise unter 50°C insbesondere unter 20-30°C liegt. Ein niedriger Schmelzpunkt des eingesetzten Phenolderivats ermöglicht die Durchführung des Verfahrens bei entsprechend niedrigen Temperaturen, was sich sehr günstig auf den Verteilungskoeffizienten $K_D$ auswirkt.

$K_D$ ist wie folgt definiert:

$$K_D = \frac{\text{Gleichgewichtskonzentration von NMP in der organischen Phase}}{\text{Gleichgewichtskonzentration von NMP in der wäßrigen Phase}}$$

Es ist auch möglich und kann vorteilhaft sein, z.B. im Hinblick auf den Schmelzpunkt, Mischungen der oben definierten Phenolderivate als Extraktionsmittel beim erfindungsgemäßen Verfahren einzusetzen.

Gemäß einer weiteren bevorzugten Ausführungsform der vorliegenden Erfindung ist das erfindungsgemäße Verfahren dadurch gekennzeichnet, daß man das Phasenvolumenverhältnis von NMP enthaltender Lösung und Alkylphenol im Bereich von 1 : 0,5 bis 1 : 10 einstellt. Das Phasenvolumenverhältnis wird erfindungsgemäß definiert durch den Quotienten aus dem Volumen der wäßrigen, NMP enthaltenden Lösung, dividiert durch das Volumen des als Extraktionsmittel eingesetzten Alkylphenols.

In einer bevorzugten Ausführungsform der vorliegenden Erfindung wird ein Phasenvolumenverhältnis von NMP enthaltender Lösung zu Alkylphenol im Bereich von 1 : 1 bis 1 : 3 eingestellt.

Aufgrund der relativ hohen Viskosität der erfindungsgemäß einzusetzenden Alkylphenole wird dem Extraktionsmittel vorzugsweise ein inertes Verdünnungsmittel zugesetzt. Dieses dient erfindungsgemäß als Mittel zur Erniedrigung der Viskosität. Bevorzugte Verdünnungsmittel im Sinne der vorliegenden Erfindung sind hochsiedende aliphatische, alicyclische und/oder aromatische, gegebenenfalls Alkylsubstituierte Kohlenwasserstoffe. Für die Siedepunkte der inerten Verdünnungsmittel gelten grundsätzlich die gleichen Kriterien wie sie oben für die Siedepunkte der erfindungsgemäß einzusetzenden Phenolderivate angegeben wurden, d.h. daß auch die eingesetzten Verdünnungsmittel oberhalb 200°C sieden sollten. Vorzugsweise liegt der Siedepunkt der Verdünnungsmittel ebenfalls mindestens ca. 20, besser mindestens 50°C oberhalb des NMP-Siedepunktes, um eine direkte destillative Abtrennung des NMP auch von dem inerten Verdünnungsmittel zu gewährleisten. Vorzugsweise setzt man daher als inertes Verdünnungsmittel hochsiedende aliphatische, alicyclische und/oder aromatische, gegebenenfalls Alkyl-substituierte Kohlenwasserstoffe mit einem Siedepunkt von mehr als 220°C, insbesondere mehr als 250°C ein.

Die einzusetzende Menge an inertem Verdünnungsmittel wird, bezogen auf die Menge an Alkylphenol, im Bereich von bis zu 50 Gew.-% eingestellt. In einer bevorzugten Ausführungsform der vorliegenden Erfindung wird das inerte Verdünnungsmittel in einer Konzentration von bis zu 20 Gew.-%, bezogen auf Alkylphenol, eingestellt.

Als bevorzugtes Extraktionsmittel wird im Sinne der vorliegenden Erfindung Nonylphenol eingesetzt, das eine außerordentlich geringe Wasserlöslichkeit aufweist (bei Raumtemperatur weniger als 1 ppm), d.h. praktisch wasserunlöslich ist. Ein weiteres bevorzugtes Extraktionsmittel im Sinne der vorliegenden Erfindung ist p-(1,1,3,3-Tetramethyl-butyl)-phenol, das auch gelegentlich als 4-tert.-Octylphenol bezeichnet wird. Mit Hilfe der bevorzugt eingesetzten Alkylphenole ist es möglich, Extraktionsausbeuten von mehr als 99,9 % zu erreichen, wobei die Fällbäder Restgehalte von weniger als 0,1 Gew.-% aufweisen. Die besten Ergebnisse werden erfindungsgemäß erzielt, wenn Alkylphenole zusammen mit inerten Verdünnungsmitteln eingesetzt werden.

Die Extraktion des NMP erfolgt am wirtschaftlichsten durch mehrstufige Gegenstromextraktion, wobei es wegen der hohen Viskosität der Alkylphenole bei Raumtemperatur zweckmäßig ist, bei Temperaturen im Bereich von 60 bis 70°C zu arbeiten oder bei niedriger Extraktionstemperatur ein inertes Verdünnungsmittel zuzusetzen. Demnach ist das erfindungsgemäße Verfahren in einer besonderen Ausführungsform durch Extraktionstemperaturen im Bereich von 2 bis zum Siedepunkt der wäßrigen Lösung, d.h. etwa 99°C anwendbar. Eine bevorzugte Ausführungsform der vorliegenden Erfindung besteht darin, daß die Extraktion des NMP bei Temperaturen im Bereich von 60 bis 70°C durchgeführt wird.

Im Anschluß an die Extraktion von NMP aus der NMP enthaltenden Lösung wird vorzugsweise das NMP direkt aus dem Alkylphenol abdestilliert und so vom Extraktionsmittel isoliert. Dementsprechend sind Alkylphenole mit Siedepunkten unterhalb des Siedepunktes von NMP für das erfindungsgemäße Verfahren weniger geeignet, obwohl auch durch eine derartige Modifikation des erfindungsgemäßen Verfahrens NMP aus wäßrigen Lösungen entfernt werden kann.

Beispiele

**Beispiel 1a:**

Ein wäßriges Fällbad der Verspinnung aromatischer Polyamide (Speiselösung) enthaltend 35 Gew.-% NMP wurde mit dem Extraktionsmittel Nonylphenol in einem Phasenvolumenverhältnis von 1 : 4 durch intensives Vermischen bei Raumtemperatur ins Gleichgewicht gesetzt. Anschließend ließ man die Phasen absitzen, trennte sie und bestimmte die Konzentration an NMP in beiden Phasen.
Die Ergebnisse sind der Tabelle zu entnehmen.

**Beispiel 1b:**

Ein wäßriges Fällbad der Verspinnung aromatischer Polyamide (Speiselösung) enthaltend 35 Gew.-% NMP wurde mit dem Extraktionsmittel Nonylphenol in einem Phasenvolumenverhältnis von 1 : 6 durch intensives Vermischen bei Raumtemperatur ins Gleichgewicht gesetzt. Man ließ die Phasen absitzen, trennte sie und bestimmte die Konzentration an NMP in beiden Phasen.
Die Ergebnisse sind in der Tabelle wiedergegeben.

**Beispiel 1c:**

Ein wäßriges Fällbad der Verspinnung aromatischer Polyamide (Speiselösung) enthaltend 35 Gew.-% NMP wurde mit dem Extraktionsmittel Nonylphenol in einem Phasenvolumenverhältnis von 1 : 10 durch intensives Vermischen bei Raumtemperatur ins Gleichgewicht gesetzt. Nach dem Absitzenlassen der Phasen wurden diese getrennt und die Konzentration an NMP in beiden Phasen bestimmt.
Die Ergebnisse sind in der Tabelle wiedergegeben.

**Beispiel 1d:**

Ein wäßriges Fällbad der Verspinnung aromatischer Polyamide (Speiselösung) enthaltend 9 Gew.-% NMP wurde mit dem Extraktionsmittel Nonylphenol in einem Phasenvolumenverhältnis von 1 : 1 durch intensives Vermischen bei Raumtemperatur ins Gleichgewicht gesetzt. Daraufhin ließ man die Phasen absitzen, trennte sie und bestimmte die Konzentration an NMP in beiden Phasen.
Die Ergebnisse sind der Tabelle zu entnehmen.

**Beispiel 1e:**

Ein wäßriges Fällbad der Verspinnung aromatischer Polyamide (Speiselösung) enthaltend 33 Gew.-% NMP wurde mit dem Extraktionsmittel Nonylphenol in einem Phasenvolumenverhältnis von 1 : 2 durch intensives Vermischen bei 70°C ins Gleichgewicht gesetzt. Nach Absitzenlassen der Phasen wurden diese getrennt und die Konzentration an NMP in beiden Phasen bestimmt.
Die Ergebnisse sind in der Tabelle wiedergegeben.

**Beispiel 1f:**

Ein wäßriges Fällbad der Verspinnung aromatischer Polyamide (Speiselösung) enthaltend 33 Gew.-% NMP wurde mit dem Extraktionsmittel Nonylphenol in einem Phasenvolumenverhältnis von 1 : 10 durch intensives Vermischen bei 70°C ins Gleichgewicht gesetzt. Dann ließ man die Phasen absitzen. trennte sie und bestimmte die Konzentration an NMP in beiden Phasen.

Die Ergebnisse sind in der nachfolgenden Tabelle wiedergegeben.

Tabelle

| Verteilungskoeffizienten für NMP im System: wäßriges Fällbad-Nonylphenol | | | |
|---|---|---|---|
| Beispiel | organische Extraktphase (Gew.-% NMP) | wäßrige Raffinatphase (Gew.-% NMP) | $K_D$ |
| 1a | 8,2 | 0,75 | 11 |
| 1b | 5,8 | 0,30 | 19 |
| 1c | 3,4 | 0,12 | 28 |
| 1d | 8,3 | 0,64 | 13 |
| 1e | 12,4 | 9,0 | 1,4 |
| 1f | 3,4 | 1,0 | 3,4 |

**Beispiel 2:**

Wäßriges Fällbad enthaltend 33,4 Gew.-% NMP wurde bei Raumtemperatur durch schubweise Gegenstromextraktion mit Nonylphenol extrahiert. Mit 4 Stufen und 1,43 Gewichtsteilen Nonylphenol pro Gewichtsteil Fällbad ließ sich ein Restgehalt von weniger als 0,01 Gew.-% NMP im Fällbad erreichen. Die Extraktionsausbeute betrug mehr als 99,97 %.

**Beispiel 3:**

Wäßriges Fällbad (S) enthaltend 33,4 Gew.-% NMP wurde bei 70°C im Gegenstrom in einem 4-stufigen Labor-Mischabsetzer der Fa. Normag mit einer Mischkammergröße von 50 ml und einer Trennkammergröße von 400 ml bei einer durchschnittlichen Verweilzeit pro Kammer von 5 Minuten mit Nonylphenol (X) beim Phasenvolumenverhältnis X : S = 4 : 1 extrahiert. Das wäßrige Raffinat enthielt weniger als 0,3 Gew-% NMP. Die Extraktionsausbeute betrug 99,4 %.

Mit gleichem Erfolg lassen sich auch andere handelsübliche Mischabsetzer einsetzen.

**Beispiel 4:**

Wäßriges Fällbad (S) enthaltend 28 Gew.-% NMP wurde bei 55°C im Gegenstrom analog Beispiel 3 mit 20 Gew.-% Diisopropylnaphthalin (Handelsprodukt Rüttgerswerke AG, Frankfurt/Main, Isomerengemisch stellungsisomerer Diisopropylnaphthaline - Siedebereiche 290 - 298 °C) in Nonylphenol extrahiert. Beim Phasenvolumenverhältnis X : S = 2,42 : 1 enthielt das wäßrige Raffinat noch 0,02 Gew.-% NMP. Die Extraktionsausbeute betrug 99,96 %.

**Beispiel 5:**

Wäßriges Fällbad (S) enthaltend ca. 35 Gew.-% NMP wurde bei 60°C mehrstufig, d.h. mit etwa 5 bis 6 theoretischen Stufen im Gegenstrom in einer Schwingbodenkolonne, System Karr, DN 25 von 2,5 m Länge mit Nonylphenol (X) extrahiert. Beim Phasenvolumenverhältnis X : S = 2 : 1 enthielt das wäßrige Raffinat noch 0,004 bis 0,002 Gew.-% NMP. Die Extraktionsausbeute betrug ca. 99,99 %.

**Beispiel 6:**

Wäßriges Fällbad (S) enthaltend ca. 35 Gew.-% NMP wurde bei 60°C mehrstufig, d.h. mit etwa 5 bis 6 theoretischen Stufen im Gegenstrom in einer Technikums-Karr-Kolonne von 4 m Länge mit 10 Gew.-% Diisopropylnaphthalin in Nonylphenol (X) extrahiert. Beim Phasenvolumenverhältnis X : S = 2 : 1 enthielt das wäßrige Raffinat noch 0,03 bis 0,05 Gew.-% NMP. (Extraktionsausbeute ca. 99,9 %).

**Beispiel 7:**

Wäßriges Fällbad (S) enthaltend ca. 35 Gew.-% NMP wurde bei 60°C im Gegenstrom in einem handelsüblichen, 6-stufigen Labor-Mischabsetzer mit 69 Gew.-% Nonylphenol in Toluol (X) extrahiert. Beim Phasenvolumenverhältnis X : S = 2 : 1 enthielt das wäßrige Raffinat weniger als 0,1 Gew.-% NMP. Ausbeute 99,7 %.

**Beispiel 8:**

Wäßriges Fällbad (S) enthaltend ca. 35 Gew.-% NMP wurde bei 60°C analog Beispiel 7 im Gegenstrom mit 71 Gew.-% Nonylphenol in Dodekan (X) extrahiert. Beim Phasenvolumenverhältnis X : S = 2 : 1 enthielt das wäßrige Raffinat noch 0,04 Gew.-% NMP. Ausbeute 99,9 %.

**Beispiel 9:**

Wäßriges Fällbad (S) enthaltend ca. 35 Gew.-% NMP wurde bei 90°C analog Beispiel 7 im Gegenstrom mit p-(1,1,3,3-Tetramethyl-butyl)-phenol (X) extrahiert. Beim Phasenvolumenverhältnis X : S = 2 : 1 enthielt das wäßrige Raffinat noch 0,04 Gew.-% NMP. Ausbeute99,9 %.

**Patentansprüche**

1. Verfahren zur Extraktion von N-Methyl-Pyrrolidon-(2) aus wäßrigen Lösungen, dadurch gekennzeichnet, daß als Extraktionsmittel bei Raumtemperatur in Wasser unlösliche oder schwerlösliche, einfach oder mehrfach durch geradkettige oder verzweigte aliphatische Reste kernsubstituierte Phenole, ggf. in Mischung mit einem inerten Verdünnungsmittel eingesetzt werden, wobei der aliphatische Substituent, oder, bei Mehrfachsubstitution, einer der aliphatischen Substituenten des Phenols eine längere Kohlenstoffkette hat, evtl. vorhandene weitere aliphatische Substituenten kurz- oder längerkettig sein können, alle aliphatischen Substituenten des Phenols gemeinsam jedoch nicht mehr als 16 C-Atome haben, und daß die wäßrigen Ausgangslösungen mindestens 25 Gew.-% N-Methyl-Pyrrolidon-(2) enthalten.

2. Verfahren nach Anspruch 1, dadurch gekennzeichnet, daß die wäßrigen Ausgangslösungen mindestens 28 Gew.-% N-Methyl-Pyrrolidon-(2) enthalten.

3. Verfahren gemäß Anspruch 1, dadurch gekennzeichnet, daß der aliphatische Substituent, oder, bei Mehrfachsubstitution einer der aliphatischen Substituenten des Phenols 4 bis 16 C-Atome, vorzugsweise 6 bis 12 C-Atome hat, evtl. vorhandene weitere aliphatische Substituenten 1 bis 8 C-Atome aufweisen können, jedoch alle aliphatischen Substituenten des Phenols gemeinsam 5 bis 16 C-Atome haben.

4. Verfahren gemäß mindestens einem der Ansprüche 1 bis 3, dadurch gekennzeichnet, daß der längerkettige aliphatische Substituent des Phenols 6 bis 12 C-Atome aufweist und bei Mehrfachsubstitution alle aliphatischen Substituenten gemeinsam 7 bis 12 C-Atome haben.

5. Verfahren gemäß mindestens einem der Ansprüche 1 bis 4, dadurch gekennzeichnet, daß die aliphatischen Substituenten des Phenols Alkylreste sind.

**Claims**

1. A process for extracting N-methyl-2-pyrrolidone from aqueous solutions, which comprises using, as the extractant, phenols which are insoluble or sparingly soluble in water at room temperature and are mono-substituted or polysubstituted in the nucleus by straight-chain or branched aliphatic radicals, if appropriate as a mixture with an inert diluent, the aliphatic substituent, or one of the aliphatic substituents in the case of polysubstitution, of the phenol having a relatively long carbon chain, it being possible for any further aliphatic substituents present to be short-chain or longer-chain, but all the aliphatic substituents of the phenol together not having more than 16 carbon atoms, and which comprises the aqueous starting solutions containing at least 25% by weight of N-methyl-2-pyrrolidone.

2. The process as claimed in claim 1, wherein the aqueous starting solutions contain at least 28% by weight of N-methyl-2-pyrrolidone

3. The process as claimed in claim 1, wherein the aliphatic substituent, or one of the aliphatic substituents in the case of polysubstitution, of the phenol has 4 to 16 carbon atoms, preferably 6 to 12 carbon atoms, any further aliphatic substituents present may have 1 to 8 carbon atoms, but all the aliphatic substituents of the phenol together have 5 to 16 carbon atoms.

4. The process as claimed in at least one of claims 1 to 3, wherein the longer-chain aliphatic substituent of the phenol has 6 to 12 carbon atoms and, in the case of polysubstitution, all the aliphatic substituents together have 7 to 12 carbon atoms.

5. The process as claimed in at least one of claims 1 to 4, wherein the aliphatic substituents of the phenol are alkyl radicals.

**Revendications**

1. Procédé pour l'extraction de la N-méthyl-pyrrolidone (2) des solutions aqueuses, caractérisé en ce que l'on utilise comme agents d'extraction des phénols substitués sur le noyau une ou plusieurs fois par des radicaux aliphatiques linéaires ou ramifiés, insolubles ou peu solubles dans l'eau à la température ambiante, éventuellement en mélange avec un diluant inerte, le substituant aliphatique ou, dans le cas de substitution multiple, un des substituants aliphatiques du phénol pouvant avoir la chaîne hydrocarbonée plus longue, d'autres substituants aliphatiques éventuellement présents pouvant avoir une chaîne courte ou une chaîne longue, toutefois tous les substituants aliphatiques du phénol ne doivent pas avoir plus de 16 atomes de carbone, et en ce que les solutions aqueuses de départ ont une teneur d'au moins 25 % en poids en N-méthyl-pyrrolidone (2).

2. Procédé selon la revendication 1, caractérisé en ce que les solutions aqueuses de départ ont une teneur d'au moins 28 % en poids en N-méthyl-pyrrolidone (2).

3. Procédé selon la revendication 1, caractérisé en ce que le substituant aliphatique ou, dans le cas de substitution multiple, un des substituants aliphatiques du phénol comporte 4 à 16 atomes de carbone, de préférence 6 à 12 atomes de carbone, d'autres substituants aliphatiques éventuellement présents pouvant présenter 1 à 8 atomes de carbone, toutefois tous les substituants aliphatiques du phénol ont ensemble 5 à 16 atomes de carbone.

4. Procédé selon au moins l'une des revendications 1 à 3, caractérisé en ce que le substituant du phénol à chaîne plus longue présente 6 à 12 atomes de carbone et, dans le cas de substitution multiple, tous les substituants aliphatiques ont ensemble de 7 à 12 atomes de carbone.

5. Procédé selon au moins l'une des revendications 1 à 4, caractérisé en ce que les substituants aliphatiques du phénol sont des radicaux alkyle.